# EUROPEAN PATENT SPECIFICATION

(11) **EP 1 078 121 B1**
(45) Date of publication and mention of the grant of the patent: **23.06.2004**
(21) Application number: 99918490.6
(22) Date of filing: 12.04.1999
(51) Int. Cl.: D06M 11/00, D06M 11/47, D06M 11/48, A61L 9/16, B01D 53/34, B01D 53/86, D06M 11/68

(54) **METHODS FOR REMOVING A CONTAMINANT BY A POLYOXOMETALATE-MODIFIED FABRIC OR A POLYOXOMETALATE-MODIFIED CELLULOSIC FIBER AND FABRICS THEREOF**
VERFAHREN ZUR ENTFERNUNG VON VERUNREINIGUNGEN MIT POLYOXOMETALATMODIFIZIERTEN GEWEBEN, ODER POLYOXOMETALATMODIFIZIERTEN CELLULOSEFASERN UND DARAUS HERGESTELLTE GEWEBE
PROCEDES D'ELIMINATION DE CONTAMINANT PAR UN TISSU MODIFIE AU POLYOXOMETALATE OU PAR UNE FIBRE CELLULOSIQUE MODIFIEE AU POLYOXOMETALATE ET TISSUS AFFERENTS

(30) Priority: 13.04.1998 US 81595 P
(43) Date of publication of application: 28.02.2001
(60) Divisional application: 04008079.8
(73) Proprietor: EMORY UNIVERSITY, Atlanta, GA 30322 (US)
(72) Inventor: HILL, Craig, L., Atlanta, GA 30345 (US); XU, Ling, Atlanta, GA 30345 (US)
(74) Representative: Leissler-Gerstl, Gabriele
(86) International application number: PCT/US1999/007889
(87) International publication number: WO 1999/053131

(56) References cited:
- US-A- 3 504 997
- US-A- 4 714 482
- ROBIN DAMICO GALL ET AL.: "Carbon Powder and Fiber-Supported Polyoxometalate Catalytic Materials. Preparation, Characterization, and Catalytic Oxidation of Dialkyl Sulfides as Mustard (HD) Analogues" CHEMISTRY OF MATERIALS., vol. 8, no. 10, 1996, pages 2523-2527, XP002111915 AMERICAN CHEMICAL SOCIETY, WASHINGTON., US ISSN: 0897-4756 cited in the application
- DATABASE WPI Section Ch, Week 9333 Derwent Publications Ltd., London, GB; Class A97, AN 93-261861 XP002111916 & JP 05 179188 A (PENTEL KK), 20 July 1993 (1993-07-20) cited in the application
- DATABASE WPI Section Ch, Week 8215 Derwent Publications Ltd., London, GB; Class A14, AN 82-30427E XP002111917 & SU 834 280 A (SARAT NITRON MFG),
- DATABASE WPI Section Ch, Derwent Publications Ltd., London, GB; Class A16, AN 71-67877S XP002111918 & JP 46 036516 B (KANEGAFUCHI SPINNING CO LTD) cited in the application

## Description

The invention relates to the use of polyoxometalate-modified fabrics and articles comprising a polyoxometalate-modified fabric for deodorizing malodorous gaseous contaminants.

### BACKGROUND OF THE INVENTION

Offensive odors originating from cigarette smoke, sweat, exhaust gases, and rotten food in the work place, the home, and elsewhere are caused by thousands of gaseous components. Examples of deleterious and/or foul-smelling compounds include, but are not limited to, acetaldehyde, hydrogen sulfide, methyl mercaptan, ammonia, trimethylamine, and nicotine.

The goal is to fabricate and use self-deodorizing fabrics and cellulosic fibers in the form of clothing, furniture upholstery, curtains, carpets, or paper to remove or degrade gaseous malodorous compounds in the work place and home. Textiles produced in Japan are known to remove contaminants from the gas phase. Some of the Japanese textiles have carbonate anions or amines incorporated within the fiber. CLEAN GUARD, which is manufactured by Komatsu Seiren Co., LTD of Japan, contains finely divided ceramics and amphoteric oxides as the deodorizing components incorporated within the fabric. Smoklin®, which is a cloth composed of polyacrylic yam manufactured by Asahi Chemical Industry Company, can also remove contaminants from the gas phase. U.S. Patent No. 5,603,927 to Fukumoto *et al.* discloses the incorporation of acid salts of aniline halides, amino benzoic acid, sulfanilamide or derivatives thereof, or aminoacetophenone into a porous carrier such as a fiber or cloth in order to remove offensive odors.

The prior art in this area, however, demonstrates a serious shortcoming, namely that the materials are effective only through stoichiometric physisorption or chemisorption processes based on acid-base, ion pairing (salt formation) and/or oxidation-reduction reactions. Because this technology is stoichiometric and not catalytic, it is not very practical and economical.

The incorporation of a polyoxometalate (herein referred to as "POM") into a fabric or cellulosic fiber in order to remove a contaminant from the gas phase has not been disclosed in the art. Furthermore, the prior art is very limited with respect to the use of a polyoxometalate-modified fabric or cellulosic fiber for the removal of a contaminant from the liquid phase. Gall *et al. (Chem. Mat.* 8, pp. 2523-2527, 1996) disclose the immobilization of H₅PV₂Mo₁₀O₄₀ on carbon cloth in order to determine the ability of H₅PV₂Mo₁₀O₄₀ to remove sulfur containing compounds from toluene. However, Gall *et al.* did not investigate the removal of sulfur containing compounds from the gas phase.

Typically, heteropoly and isopoly acids, which are subsets of polyoxometalates, are used as pigments and dyes when they are incorporated into a fabric. Japanese Patent Application No. JP 50136488 to Kakinuma *et al.* discloses contacting yarn with a heteropoly acid or tartaric acid to improve the lightfastness of the yarn. Japanese Patent No. JP 82014477 B discloses the lightfastness of yarn is improved when the yarn is contacted with an aqueous solution of phosphomolybdic acid.

JP 5179188, JP 61185568, and JP 62013464 disclose that heteropoly acids such as phosphotungstic acid, phosphomolybdic acid, silicomolybdic acid, silicotungstic acid, phosphotungstomolybdic acid, phosphovanadomolybdic acid, and their salts can be used as colorants and pigments for ink compositions. U.S. Patent No. 3,947,332 to Vanderpool *et al.* disclose the synthesis of heteropoly acids containing tungsten or molybdenum and at least one other element having a positive valence from 2 to 7. The heteropoly acids disclosed in Vanderpool *et al.* can be used in printing inks and paper coloring.

U.S. Patent No. 3,925,006 to Forschirm *et al.* discloses first contacting a cellulose ester with sodium tungstophosphate or sodium tungstosilicate followed by contacting the cellulose ester with a cationic dye. The sodium tungstophosphate and sodium tungstosilicate improve the uptake of the cationic dye into the cellulose ester. U.S. Patent No. 4,444,592 to Ludwig discloses the preparation of a pigment that is the reaction product between a heteropoly acid and pararosaniline. The water insoluble pigment can be used in the textile arts. *Chem. Rev.* 1998, 98, pp. 359-387 to Katsoulis is a comprehensive review article that discloses the use of POMS as dyes and pigments.

Japanese Patent No. JP 71036516 B discloses a method for resin-treating textile goods containing cellulosic or hydrophobic fibers. The cellulosic fiber is treated with an isopoly or heteropoly acid. The resultant cellulosic fiber possesses increased soil-and-crease resistance and wash-and-wear properties. International Patent Application No. 94/20565 to Jackson *et al.* discloses contacting an aramid with an aqueous solution of a tungsten compound, preferably phosphotungstic acid and ammonium metatungstate, in order to decrease the flammability of textile articles. None of the references described above disclose the removal of a contaminant from the gas or liquid phase.

A POM-based fixed-bed catalytic reactor for the purification of gas in vents or passages has been disclosed in Japanese Patent Application No. 0435716 to Akio *et al.* This reactor involves a POM (H₃PMo₆W₆O₄₀) immobilized on a porous moulding of cordierite.

Japanese Patent Application No. 7251075 discloses the gas phase oxidation of unsaturated aldehydes to the corresponding carboxylic acid by a heteropoly acid catalyst containing molybdenum and vanadium. The catalyst is used in combination with carbon fibers. There is no disclosure in JP 7251075, however, for the removal of a contaminant from the gas or liquid phase.

In light of the above, it would be very desirable to provide a use of an article for the removal of malodorous compounds without adding stoichiometric amounts of additives or compounds to the article. The present invention solves such a need in the art while providing surprising advantages. The present invention relies on the use of a catalytically active early-transition-metal oxygen anion cluster (polyoxometalate or POM) incorporated into textiles, fabrics, and cellulosic fibers, including the Japanese deodorizing fabrics described above, which greatly increases the ability of the fabric to remove odorous compounds from the gas phase.

### SUMMARY OF THE INVENTION

The invention relates to the use of a polyoxometalate-modified fabric, comprising a fabric and at least one polyoxometalate, wherein the polyoxometalate has the formula [VₖMoₘWₙNbₒTaₚM_{q}XᵣOₛ]^{y-}[A], wherein M is at least one f-block element or d-block element having at least one d-electron, wherein M is not vanadium, molybdenum, tungsten, niobium, or tantalum; X is at least one p-, d-, or f-block element, wherein X is not oxygen; k is from 0 to 30; m is from 0 to 160; n is from 0 to 160; o is from 0 to 10; p is from 0 to 10; q is from 0 to 30; r is from 0 to 30; s is sufficiently large that y is greater than zero; and y is greater than zero, wherein the sum of k, m, n, o, and p is greater than or equal to four; and the sum of k, m, and q is greater than zero, and A is a counterion, wherein the polyoxometalate is incorporated in the fabric,
with the proviso that when A is a proton, the polyoxometalate is not the reaction product between [VₖMoₘWₙNbₒTaₚM_{q}XᵣOₛ]^{y-}[A] and a pararosaniline compound,
with the further proviso that the polyoxometalate is not silicomolybdenic acid or its sodium salt, phosphomolybdenic acid, ammonium chromododecanemolybdenate, ammonium salt of hydrogen hexamolybdocobaltic acid, para-tungstic acid or its ammonium salt or sodium salt, meta-tungstic acid or its ammonium salt or sodium salt, phosphotungstic acid or its salt, silicotungstic acid or its salt, dodecane tungstodicobaltic acid or its salt, phosphotungstomolybdenic acid or its salt, or phosphovanadomolybdenic acid or its salt,
with the further proviso that when the fabric is carbon cloth, the polyoxometalate is not H₅PV₂Mo₁₀O₄₀.

Additional advantages of the invention will be set forth in part in the description which follows, and in part will be obvious from the description, or may be learned by practice of the invention. The advantages of the invention will be realized and attained by means of the elements and combinations.

### BRIEF DESCRIPTION OF THE DRAWINGS

Figure 1 shows the DRIFT spectra of 1-acrylic (5 wt% of 1, bottom) and unsupported 1 powder (top), where is H₅PV₂Mo₁₀O₄₀.
Figure 2 shows the SEM pictures of acrylic (top) and 1 -acrylic (5 wt% of 1, bottom).
Figure 3 shows the aerobic oxidation of gaseous CH₃CHO by Smoklin® and Smoklin® with adsorbed 1.

### DETAILED DESCRIPTION OF THE INVENTION

The present invention may be understood more readily by reference to the following detailed description and the Examples included therein.

In this specification and in the claims which follow, reference will be made to a number of terms which shall be defined to have the following meanings:
The singular forms "a," "an" and "the" include plural referents unless the context clearly dictates otherwise.
"Optional" or "optionally" means that the subsequently described event or circumstance may or may not occur, and that the description includes instances where said event or circumstance occurs and instances where it does not.

In accordance with the purpose(s) of this invention, as defined in the claims, as embodied and broadly described herein, this invention, in one aspect, relates to the use of a polyoxometalate-modified fabric for deodorization by removing or degrading gaseous malodorous contaminants from the gas phase, comprising contacting an article comprising a fabric and at least one polyoxometalate, wherein the polyoxometalate is incorporated in the fabric with the gas phase containing the contaminant.

The invention further relates to the use of an article comprising a cellulosic fiber and at least one polyoxometalate, wherein the polyoxometalate is incorporated in the cellulosic fiber to produce a polyoxometalate-modified cellulosic fiber, for removing gaseous compounds as defined in claim 17.

Many polyoxometalates known in the art can be used in the present invention to remove a contaminant from the gas phase. Polyoxometalates are also referred to in the art as heteropoly compounds, heteropoly acids, isopoly compounds, and isopoly acids, which are subsets of polyoxometalates. Examples of polyoxometalates useful in the present invention are disclosed in Pope, M.T. in *Heteropoly and Isopoly Oxometalates,* Springer Verlag, 1983, and *Chemical Reviews,* vol. 98, no. 1, pp. 1-389, 1998, which are incorporated by this reference in their entirety.

The selection of the polyoxometalate used in the present invention is dependent upon the contaminant or contaminants to be removed from the gas phase phase. In one embodiment, the polyoxometalate has the formula [VₖMoₘWₙNbₒTaₚM_{q}XᵣOₛ]^{y-}[A], wherein M is at least one f-block element or d-block element having at least one d-electron, wherein M is not vanadium, molybdenum, tungsten, niobium, or tantalum; X is at least one p-, d-, or f-block element, wherein X is not oxygen; k is from 0 to 30; m is from 0 to 160; n is from 0 to 160; o is from 0 to 10; p is from 0 to 10; q is from 0 to 30; r is from 0 to 30; s is sufficiently large that y is greater than zero; and y is greater than zero, wherein the sum of k, m, n, o, and p is greater than or equal to four; and the sum of k, m, and q is greater than zero, and A is a counterion. In one embodiment, s is from 19 to 460. The charge on the POM, y, is dictated by the values of k, m, n, o, p, q, r and s. The p-, d-, and f-bock elements can exist in any oxidation state.

The counterion A can be any counterion known in the art. Examples of counterions include, but are not limited to, quaternary ammonium cation, proton, alkali metal cation, alkaline earth metal cation, ammonium cation, d-block cations, f-block cations, or a combination thereof. In one embodiment, the polyoxometalate is an acid, wherein the counterion A is H⁺. In one embodiment, the counterion is a d- or f-block metal complex. In one embodiment, the counterion is trimethyl-triazacyclononane manganese. In another embodiment, the counterion is silver or gold. Not wishing to be bound by theory, it is believed that some counterions of the present invention can be reduced to the corresponding metal when the polyoxometalate contacts the contaminant. For example, when the cation is Ag⁺¹ or Au⁺¹, these cations can be reduced to silver metal or gold metal, respectively, depending upon the contaminant that is to be removed.

Generally, M can be any d-block element having at least one d-electron. Typically, M comprises titanium, chromium, manganese, cobalt, iron, nickel, copper, rhodium, silver, palladium, platinum, mercury, or ruthenium. In a preferred embodiment, M comprises manganese, cobalt, or ruthenium. In another embodiment, X comprises phosphorus, silicon, aluminum, boron, cobalt, zinc, or iron. When the polyoxometalate has the Keggin structure XM₁₂, then it is possible for X and at least one M to be the same d- or f-block element. Not wishing to be bound by theory, it is believed that the metal ion M of the polyoxometalate of the present invention is responsible for removing the contaminant from the gas phase, while X, when present, provides structural integrity to the polyoxometalate.

In one embodiment, the sum of k and q is greater than or equal to one, the sum ofk, m, n, o, p, and q is 12, and s is 40. In yet another embodiment, k is not zero. In another embodiment, q is not zero. In a preferred embodiment, the polyoxometalate comprises H₅PV₂Mo₁₀O₄₀, H₄PVMo₁₁O₄₀, H₆PV₃Mo₉O₄₀, Na₉PV₆Mo₆O₄₀, Na₅H₂PV₄Mo₈O₄₀, or K₈Co₂W₁₁O₃₉.

In another embodiment, the polyoxometalate has the formula [X^{g+}V_{b}M^{h+}_{c}Z_{12-b-c}O₄₀]^{u-}[A], wherein X is at least one p-, d-, or f-block element; g+ is the charge of X; M is at least one f-block element or d-block element having at least one d-electron, wherein M is not vanadium; h+ is the charge of M; Z is tungsten, molybdenum, niobium, or a combination thereof; b is from 0 to 6; c is from 0 to 6, wherein the sum of b and c is greater than or equal to one; u is greater than 3; and A is a counterion.

In another embodiment, the polyoxometalate has the formula [X^{g+}V_{b}Z_{12-b}O₄₀]^{u-}[A], wherein X is at least one phosphorus, silicon, aluminum, boron, zinc, cobalt, or iron; Z comprises tungsten, molybdenum, niobium, or a combination thereof; b is from 1 to 6; and u is greater than 3.

In another embodiment, the polyoxometalate has the structure (X^{g+}M^{h+}_{c}Z_{12-c}O₄₀]^{u-}[A], wherein X is at least one phosphorus, silicon, aluminum, boron, zinc, cobalt, or iron; Z comprises tungsten, molybdenum, niobium, or a combination thereof; M^{h+} is at least one [f-block element or d-block element having at least one d-electron; c is from 1 to 6; and u is greater than 3.

In another embodiment, the polyoxometalate has the formula [Xⁱ⁺₂VᵤM^{j+}ᵥZ₁₈₋ᵤ₋ᵥO₆₂]^{w-}[A], wherein X is at least one p-, d-, or f-block element; i+ is the charge of X; M is at least one d- or f-block element, wherein M is not vanadium; j+ is the charge of M; Z is tungsten, molybdenum, niobium, or a combination thereof; u is from 0 to 9; v is from 0 to 9, wherein the sum of u and v is greater than or equal to one; w is greater than or equal to 4; and A is a counterion.

In another embodiment, the polyoxometalate has the formula [Xⁱ⁺₂VᵤZ₁₈₋ᵤO₆₂]^{w-}[A], wherein X is at least one phosphorus, sulfur, silicon, aluminum, boron, zinc, cobalt, or iron; Z comprises tungsten, molybdenum, niobium, or a combination thereof; u is from 1 to 9; and w is greater than or equal to 4.

In another embodiment, the polyoxometalate has the formula [Xⁱ⁺₂M^{j+}ᵥZ₁₈₋ᵥO₆₂]^{w-}[A], wherein X is at least one phosphorus, sulfur, silicon, aluminum, boron, zinc, cobalt, or iron; Z comprises tungsten, molybdenum, niobium, or a combination thereof; M^{j+} is at least one d- or f-block element; v is from 1 to 9; and w is greater than or equal to 4.

In another embodiment, the polyoxometalate has the formula [YVₓZ₁₂₋ₓO₄₀][A], wherein Y is phosphorus, silicon, or aluminum; Z is tungsten or molybdenum; x is from 1 to 6, and A is a counterion. In one embodiment, Y is phosphorus and Z is molybdenum. In one embodiment, Y is phosphorus and Z is tungsten. In one embodiment, Y is silicon and Z is molybdenum. In one embodiment, Y is silicon and Z is tungsten. In one embodiment, Y is aluminum and Z is tungsten. In one embodiment, Y is aluminum and Z is molybdenum.

Polyoxometalates having an organic group, such as an alkyl group or aryl group, an organosilyl group, or other p- or d-block organometallic groups bonded to the POM can also be used in the present invention. The organic group can be branched or straight chain alkyl, alkenyl, or alkynyl group or an aryl group of C₁ to C₃₀. The alkyl group can also be a polyether or polyol. Not wishing to be bound by theory, the organic group is bonded to the polyoxometalate as depicted in Scheme 1, where R is the organic group:
The reaction between an alcohol and the polyoxometalate I results in the loss of water and the formation of the polyoxometalate II, wherein the organic group is bonded to an oxygen atom of the polyoxometalate. Any alcohol known in the art can be used in the present invention. Examples of alcohols that can be used include, but are not limited to, methanol, ethanol, or tris(hydroxymethyl)methane. The polyoxometalates having organic groups bonded to the POM that are disclosed in Gouzerh *et al., Chem. Rev.,* 98, pp. 77-111, 1998, which is incorporated by reference in its entirety, are useful in the present invention.

In another embodiment, the polyoxometalate I can be reacted with a compound having the generic formula YAₒR₄₋ₒ, wherein Y is silicon, tin, or an other p- or d-block element; A is a leaving group; R is an organic group, such as an alkyl, alkenyl, or alkynyl group or an aryl group of C₁ to C₃₀; and o is from 1 to 4. Suitable leaving groups for A include, but are not limited to, halides and alkoxides. In Scheme I, the oxygen of polyoxometalate I displaces A from YAR₃ to form a new Y-O bond (compound III). Any silyl, tin, or organic derivative of a p- or d-block element known in the art can be used in the present invention, provided that the compound has at least one leaving group. Typically, Met in Scheme I is vanadium, molybdenum, tungsten, niobium, or tantalum.

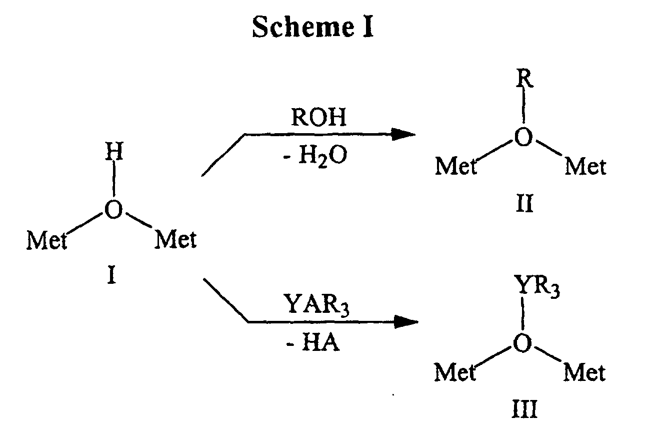

A POM can be incorporated into any fabric known in the art to produce a polyoxometalate-modified fabric useful according to the present invention. In one embodiment, fabrics used to prepare garments, draperies, carpets, and upholstery can be used and articles made from them are a part of this invention. In another embodiment, the fabric can be a knit or non-woven fabric. Fibers useful in preparing the polyoxometalate-modified fabrics include, but are not limited to, polyamide, cotton, polyacrylic, polyacrylonitrile, polyester, polyvinylidine, polyolefin, polyurethane, polytetrafluoroethylene, or carbon cloth, or a combination thereof. In one embodiment, the fabric is prepared from cotton, polyacrylic, or polyacrylonitrile. In one embodiment, the fabric is prepared from a cationic fiber. In another embodiment, the fabric comprises (1) a 50/50 blend of nylon-6,6 and cotton or (2) stretchable carbon blended with polyurethane.

The amount of polyoxometalate incorporated in the fabric also varies depending upon the contaminant to be removed. There is no restriction on the amount of POM that can be incorporated into the fabric. In one embodiment, the amount of polyoxometalate incorporated in the fabric is from 2.5 to 60%, 2.5 to 35%, 2.5 to 30%, 2.5 to 25%, 2.5 to 20%, and 2.5 to 15% based on the total weight of the polyoxometalate-modified fabric.

In one embodiment, the polyoxometalate is H₅PV₂Mo₁₀O₄₀ and the fabric is prepared from a polyacrylic fiber. In another embodiment, the polyoxometalate is H₅PV₂Mo₁₀O₄₀ and the fabric is prepared from cotton.

The present invention is capable of removing a single contaminant or multiple contaminants from the gas phase. The term "remove" refers to, but is not limited to, the degradation of the contaminant, the conversion of the contaminant into another compound that is less malodorous, or the adsorption of the contaminant by the polyoxometalate. The POM can degrade the contaminant by a number of different mechanisms. For example, the POM can aerobically oxidize the contaminant acetaldehyde (CH₃CHO). Not wishing to be bound by theory, it is believed that the aerobic oxidation of CH₃CHO proceeds by a radical chain mechanism (*i.e.*, the initiation of the radical chain by CH₃CHO + POMₒₓ -> CH₃CO^{.} + HPOM_{red}).

Contaminants that can be removed by using the present invention include, but are not limited to, aliphatic nitrogen compounds such as amines and ammonia; sulfur-containing compounds such as hydrogen sulfide, thiols, thiophenes, and thioethers; halogenated compounds; and aliphatic oxygenated compounds, such as aldehydes, ketones, organic acids, and alcohols. In one embodiment, the contaminant is acetaldehyde, methyl mercaptan, ammonia, hydrogen sulfide, methyl sulfide, dimethyl sulfide, dimethyl disulfide, trimethylamine, styrene, propionic acid, n-butyric acid, n-valeric acid, iso-valeric acid, or a combination thereof.

The present invention can remove malodorous gaseous contaminants from the gas phase under mild conditions. In one embodiment, the contaminant can be removed at from -50 °C to 105 °C at a pressure of from 0.1 to 30 atm, preferably from 25 °C to 105 °C at 1 atm. In a preferred embodiment, the present invention can remove a contaminant from the gas phase at room temperature (approximately 25 °C) and at 1 atm. These conditions are very mild when compared to industrial catalysts, which require much higher reaction temperatures and pressures in order to promote catalytic activity. In another embodiment, the present invention can remove a contaminant from the gas phase that has a partial pressure of from 0.1 ppb to 2 atm, 10 ppb to 2 atm, 100 ppb to 2 atm, 200 ppb to 2 atm, and 0.5 ppm to 2 atm. Similarly, the present invention can remove a contaminant from the liquid phase under mild conditions. In one embodiment, the contaminant can be removed from a liquid media at from 0 °C to 200 °C. The temperature depends upon the liquid media that is being contacted and the contaminant to be removed.

The gas phase containing the contaminant can be contacted by the polyoxometalate-modified fabric or cellulosic fiber using a variety of techniques. For example, the polyoxometalate-modified fabric or cellulosic fiber is typically placed in an open or closed environment that contains the contaminant(s).

The POM modified fabrics and cellulosic fibers of the present invention have a number of advantages over the prior art fabrics and fibers that do not use a polyoxometalate to remove a contaminant from the gas or liquid phase. One advantage is that the POM modified fabrics and cellulosic fibers are significantly more effective in deodorization when compared to a fabric or fiber that is not modified with a POM of the present invention. Another advantage is that the present invention can remove a contaminant from the gas or liquid phase starting within milliseconds of contact and can remove the contaminant for extended periods of time, such as several days or longer. The POMs used in the present invention are capable of being regenerated to an active form that permits the removal of the contaminant. Another advantage is that some POMs can render the fabric or cellulosic fiber more water resistant and increase the surface area of the fabric or fiber. Finally, the POM can enhance the dyeability, light fastness, color fastness, and weaving properties of the fabric or cellulosic fiber.

The invention further relates to the use of a polyoxometalate-modified fabric, comprising a fabric and at least one polyoxometalate, wherein the polyoxometalate has the formula [VₖMoₘWₙNbₒTaₚM_{q}XᵣOₛ]^{y-}[A], wherein M is at least one f-block element or d-block element having at least one d-electron, wherein M is not vanadium, molybdenum, tungsten, niobium, or tantalum; X is at least one p-, d-, or f-block element, wherein X is not oxygen; k is from 0 to 30; m is from 0 to 160; n is from 0 to 160; o is from 0 to 10; p is from 0 to 10; q is from 0 to 30; r is from 0 to 30; s is sufficiently large that y is greater than zero; and y is greater than zero, wherein the sum of k, m, n, o, and p is greater than or equal to four; and the sum ofk, m, and q is greater than zero, and A is a counterion, wherein the polyoxometalate is incorporated in the fabric,
with the proviso that when A is a proton, the polyoxometalate is not the reaction product between [VₖMoₘWₙNbₒTaₚM_{q}XᵣOₛ]^{y-}[A] and a pararosaniline compound,
with the further proviso that the polyoxometalate is not silicomolybdenic acid or its sodium salt, phosphomolybdenic acid, ammonium chromododecanemolybdenate, ammonium salt of hydrogen hexamolybdocobaltic acid, para-tungstic acid or its ammonium salt or sodium salt, meta-tungstic acid or its ammonium salt or sodium salt, phosphotungstic acid or its salt, silicotungstic acid or its salt, dodecane tungstodicobaltic acid or its salt, phosphotungstomolybdenic acid or its salt, or phosphovanadomolybdenic acid or its salt,
with the further proviso that when the fabric is carbon cloth, the polyoxometalate is not H₅PV₂MO₁₀O₄₀ for deodorization by removing or degrading malodorous gaseous contaminants.

In one embodiment, k is greater than or equal to one.

A pararosaniline compound is a compound having the structure IV 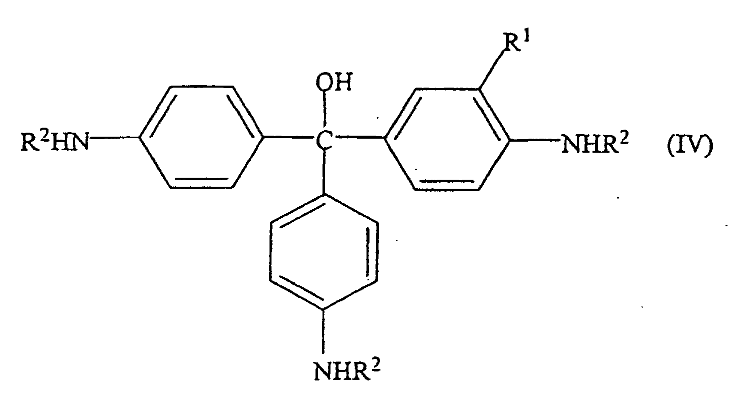 wherein R¹ is hydrogen or methyl and each R² is, independently, hydrogen or substituted or unsubstituted aryl. Examples of pararosaniline compounds not used in the present invention are disclosed in U.S. Patent No. 4,444,592 to Ludwig, which is herein incorporated by reference in its entirety.

The polyoxometalate can be incorporated into the fabric using techniques known in the art. Examples of techniques that can be used for incorporating the POM into a fabric fiber include, but are not limited to, depositing the POM on the surface of an existing fabric, covalently bonding the POM to the fibers of the fabric, impregnating or intimately mixing the POM with the fabric, electrostatically bonding the POM to the fabric, or datively bonding the POM to the fabric via the coordination of a d- or f- block metal ion on the surface of the POM with a functional group on the fabric. In the case of electrostatically bonding the POM to the fabric, the positively charged functional groups on the fabric or cellulosic fiber and the negatively charged POM can form an electrostatic bond. In one embodiment, when the counterion of the polyoxometalate is a proton, the fabric can be protonated by the polyoxometalate to produce a positively charged fiber, which then electrostatically bonds to the polyoxometalate anion. In one embodiment, a cationic polymer can be used as a binding agent to incorporate an anionic polyoxometalate into an anionic fiber.

The polyoxometalates can be incorporated in the fabric using a variety of techniques known in the art. In one embodiment, the fabric is contacted with a mixture comprising the polyoxometalate and a solvent. The polyoxometalate can be soluble, partially soluble, or insoluble in the solvent, depending upon the polyoxometalate and solvent selected. In one embodiment, the solvent is water. In another embodiment, the solvent can be an organic solvent. Examples of organic solvents useful in the present invention include, but are not limited to, acetonitrile, acetone, toluene, carbon dioxide, xylenes, 1-methyl-2-pyrrolidinone, dimethyl sulfoxide, or an alcohol, such as methanol, ethanol, 1-propanol, or 2-propanol. In another embodiment, the solvent can be used in supercritical drying technology known in the art, which results in a finely-divided, high surface-area deposited polyoxometalate on the fabric

In one embodiment, the mixture is from 0.1 to 20% by weight polyoxometalate and from 80 to 99.9% by weight water, preferably from 0.3 to 15% by weight polyoxometalate and 85 to 99.7% water. Generally, the fabric is dipped or immersed into the mixture containing the POM for several hours to days at a temperature of from 0 °C to 100 °C, preferably for 2 hours to 2 days at from 25 °C to 80 °C. In another embodiment, the POM can be admixed with a resin or adhesive, and the resultant adhesive is applied to the surface of or admixed with the fabric.

Typically, once the fabric has been contacted with the POM, the polyoxometalate-modified fabric or cellulosic fiber is dried in order to remove residual solvent. In one embodiment, the polyoxometalate-modified fabric is heated from 0 °C to 220 °C at or below atmospheric pressure, preferably from 25 °C to 100 °C. In another embodiment, the polyoxometalate-modified fabric is dried *in vacuo (i.e.,* less than or equal to 10 torr). In yet another embodiment, the polyoxometalate-modified fabric is dried under supercritical conditions.

### EXAMPLES

Efforts have been made to ensure accuracy with respect to numbers (*e.g.*, amounts, temperature, etc.) but some errors and deviations should be accounted for. Unless indicated otherwise, parts are parts by weight, target odorants are expressed in parts per million, temperature is in °C or is at ambient temperature and pressure is at or near atmospheric.

The term "consumption" refers to the removal or adsorption of a contaminant or contaminants from the gas phase or the conversion of the contaminant or contaminants to another compound(s) that is less malodorous or not malodorous.

### General Considerations

**Materials.** The polyoxometalate H₅PV₂Mo₁₀O₄₀ (**1**), H₄PVMo₁₁O₄₀ (**2**), and H₆PV₃Mo₉O₄₀ (**3**) were synthesized and purified following the procedure disclosed in Pettersson *et al., Inorg. Chem.* 1994, 33, pp. 982, and Tsigdinos *et al., Inorg. Chem.,* 1968, 7, pp. 437-441. ⁵¹V and ³¹P NMR were used to check the purity. The samples of Smoklin® (the brand name of a cloth manufactured by Asahi Chemical Industry Company, Japan, made mainly from polyacrylic yarn), acid-dyeable polyacrylic fiber and the "deep-dye" nylon fiber with NH₂ groups on the polymer ends were tested. The samples of cotton cloth were cut from a common 100% cotton T shirt after laundering. The samples of paper were obtained from Fisher Scientific. All samples were submitted to vacuum and then heated, or washed and then submitted to vacuum and then heated before use. All solvents used were purchased from Fisher or Burdick & Jackson company and had purities of greater than 99%. Acetaldehyde was obtained from Aldrich and exhibited purities greater than 99.9%. Argon, helium, and oxygen were ordered from Specialty Gases (greater than 99.5%). The gases ethane (Matheson), methyl mercaptan (Matheson) and ammonia (Air Products) were all of greater than 99% purity. All chemicals purchased were used as received, except acetaldehyde which was protected under Ar in the freezer to minimize adventitious autoxidation (radical chain oxidation by O₂) or other possible reactions.

**Instrumentation.** All reactions involving CH₃CHO and CH₃SH were monitored using a Hewlett-Packard 5890 gas chromatograph equipped with flame ionization detectors and a HP-PLOT Q divinyl benzene/styrene porous polymer capillary column. The consumption of NH₃ was detected by Sensidyne® /Gastec detector tube systems. ⁵¹V NMR measurements were carried out on a 500-MHZ General Electric GN500 Spectrometer. ³¹P NMR measurements were carried out on a 400-MHZ Varian Inova 400 Spectrometer. The diffuse reflectance infrared fourier transform spectroscopy (DRIFT) measurements for powdered H₅PV₂Mo₁₀O₄₀ and fabric systems were conducted using a Nicolet 510 FT-IR spectrometer. The scanning electron microscopy (SEM) experiments were carried out on a *ISI* DS-130/LaB₆EM. Elemental analysis were provided by E+R Microanalytical Laboratory, Inc.

**Preparation of Fabrics with Supported H**_{**5**}**PV**_{**2**}**Mo**_{**10**}**O**_{**40**} **(1). 1**-Smoklin® cloth and **1**-cotton cloth were prepared as follows: a 2-g sample of cloth was immersed in various aqueous solutions of **1** at room temperature. After 24 h impregnation, the cloth was dried at room temperature first in the air for 2 days and then *in vacuo* for another 2 days. Preparation for **1**-acrylic and **1**-nylon fibers: the nylon and acrylic fibers were washed in boiling 2-propanol for 4 h and then dried in vacuo at 323 K before use. Representative 2 g samples were then immersed in various aqueous solutions of **1** at 353 K for 2 h and then at room temperature for 1 day. Most of the water or excess solution was removed using a rotary evaporator or by filtration at room temperature. The **1**-nylon or **1**-acrylic samples were then dried at room temperature first in the air overnight and then in vacuo for 2 days. The percentages of **1** on the cloth or fibers were determined by both weight difference and elemental analysis (by E+R Microanalytical Laboratory, Inc.).

**Comparison of Fabrics Unmodified or Modified with 1 in the Aerobic Oxidation of CH**_{**3**}**CHO and CH**_{**3**}**SH**. In all reactions, 0.5 g of **1**-modified or unmodified cloth or fiber was hung in a 250-mL round-bottom flask covered with aluminum foil and sealed with a septum stopper. This apparatus was taken through three degas/gas cycles with Ar, evacuated and then purged with O₂. Next, 25 µl of gaseous CH₃CHO (or 50 µl CH₃SH) and 25 µl C₂H₆ (internal standard) were added to the flask to start the reaction. The reactions were stirred using a digital stirrer at 1200 rpm and maintained at room temperature under 1 atm of O₂. For the oxidation of CH₃SH, reactions were carried out in three steps. After 20 h of the above reaction or no detectable CH₃SH remaining in gas phase (the first step), an additional 500 µl CH₃SH was then added to the apparatus to continue the reaction in the second step. An additional 500 µl CH₃SH was added to the apparatus to initiate the reaction in the third step after 20 hr reaction of the second step. In all reactions, gaseous aliquots (1-mL) were extracted at various times and analyzed by GC. Blank control reactions with the materials (modified or unmodified) absent from the otherwise identical reaction systems were run at the same time. All reactions were run in duplicate to assess reproducibility and the averages reported.

**Evaluation of Reusability of 1-Modified Fabrics in the Aerobic Oxidation of CH**_{**3**}**CHO**. The reactions were run as described above. The **1**-fabrics were recovered by treating the fabrics *in vacuo* at room temperature for 2 days. The reactions involving these materials were then run again under the conditions identical to those in the first run.

**Comparison of Fabrics Unmodified or Modified with 1 in the Removal of NH**_{**3**}**.** In all reactions, 0.5 g of **1**-modified or unmodified cloth or fiber was hung in a 5-L round-bottom flask sealed with a septum stopper. This apparatus was taken through three degas/gas cycles with Ar. Next, 1-mL of gaseous NH₃ was injected into the flask to start the reaction. The reactions were stirred using a digital stirrer at 500 rpm and maintained at room temperature under 1 atm of Ar. After 1 hr reaction, 100-mL gaseous aliquots were extracted by the detector tube to analyze the concentration of the remaining NH₃. All reactions were run in duplicate.

**Evaluation of Fabrics Unmodified or Modified with 1 in the Deodorization of a Simulated Polluted Atmosphere (CH**_{**3**}**CHO, CH**_{**3**}**SH, and NH**_{**3**}**).** The reaction apparatus was prepared following almost the same procedure as described above, except that 500-mL flask was used. C₂H₆ (50 µl), CH₃CHO (50 µl), CH₃SH (500 µl) and NH₃ (500 µl) were injected simultaneously into the apparatus to start the reaction. The reactions were stirred using a digital stirrer at 1,200 rpm and maintained at room temperature under 1 atm of O₂. All reactions were run in duplicate to assess reproducibility and the averages reported.

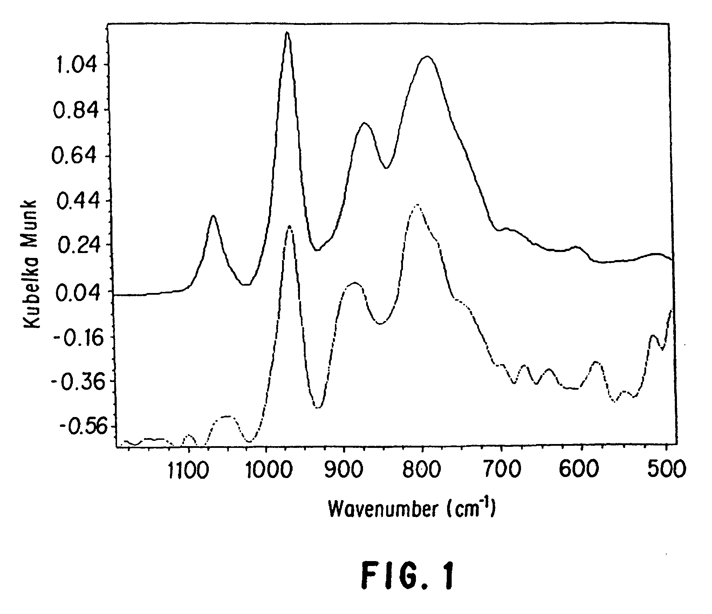

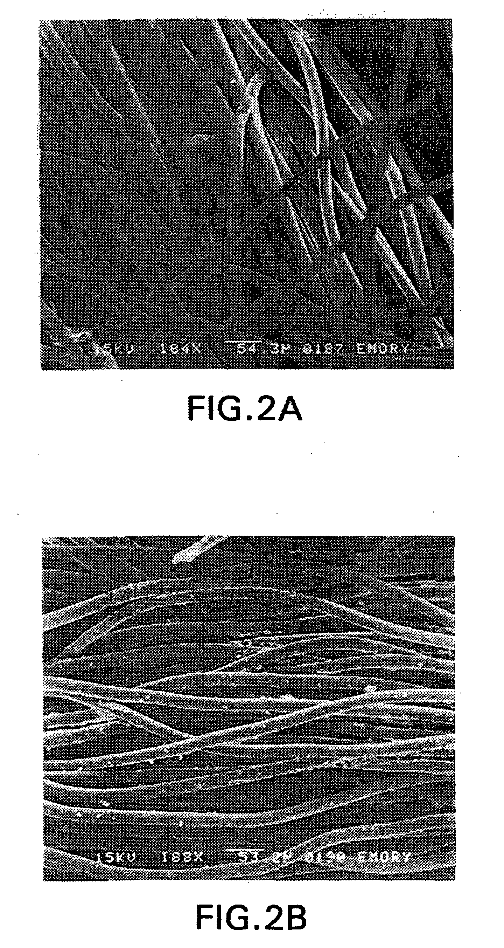

**Characterization.** The DRIFT spectra of H₅PV₂Mo₁₀O₄₀ (1) modified Smoklin® , cotton cloth, acrylic fiber and nylon fiber are all similar to that of the unsupported H₅PV₂Mo₁₀O₄₀ powder in the typical IR range (600-1200 cm⁻¹) for polyoxometalates, indicating that **1** is present on the surface of all the modified fabrics (see Figure 1, 5wt% **1**-acrylic fiber). The SEM pictures in Figure 2 demonstrate that clusters of **1** are formed unevenly on the surface of the fabrics during the preparation of **1**-acrylic. The size of most clusters of **1** on **1**-Smoklin® (5 wt%) and **1**-acrylic (5 wt%) are in the 1-5 µm range.

**Gas Phase Reactions.** In all gas phase reactions, the overall observed consumption of the reagents reflects both the reactions of the reagents and their adsorption on the glass and cloth surfaces.

### Example 1

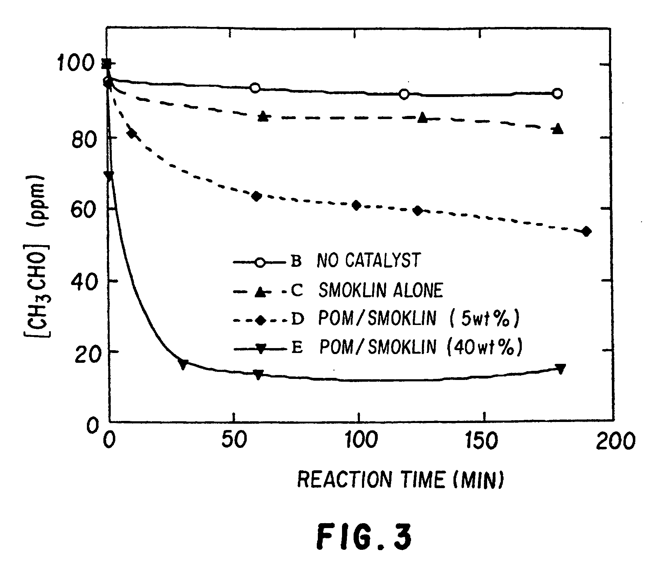

**Comparison of Catalytic Efficiencies of the 1-Fabrics in the Aerobic of Oxidation CH**_{**3**}**CHO.** All gas phase reactions were run at room temperature and under 1 atm O₂ pressure. Figure 3 displays the consumption (concentration versus time) curves when no catalyst, Smoklin® alone and **1**-Smoklin® (with both 5 wt% and 40 wt% of **1**) were used under otherwise identical reaction conditions. A comparison of the curves for the Smoklin® alone and the catalyst-free control experiment indicates that the Smoklin® has no significant activity. For the Smoklin® modified by **1**, significant activity is observed even when the weight percentage of 1 is as low as 5 wt%. Additionally, Figure 3 reveals that **1**-Smoklin® removes a contaminant from the gas phase at a much higher rate than unmodified Smoklin®, which is another advantage of the present invention.

Table 1 summarizes the results for different cloth and fiber systems in 3 hr reactions. All the unmodified fabrics, the **1**-nylon (even when the present of 1 is as high as 15 wt%) and the 1-cotton cloth (5 wt% of 1) exhibit little or no activity. In contrast, significant activity is demonstrated for the **1**-Smoklin® (5 wt% of **1**), the **1**-acrylic fiber (5 wt% of **1**) and the **1**-cotton cloth (15 wt% of **1**) with no detectable CH₃COOH present in gas phase.

**Table 1.**

| Removal of CH₃CHO from the Gas Phase by Polyoxometalate-Modified Fabrics.^{*a*} | |
|---|---|
| Fabric | Consumption of CH₃CHO (%)^{*b*} |
| None^{*c*} | 9 |
| | |
| Smoklin® | 15 |
| **1**-Smoklin® (5 wt% **1**) | 49 |
| **1**-Smoklin® (10 wt% **1**) | 55 |
| **1**-Smoklin® (40 wt% **1**) | 89 |
| | |
| acrylic | 13 |
| **1**-acrylic (5 wt% **1**) | 62 |
| | |
| cotton | 26 |
| **1**-cotton (5 wt% **1**) | 30 |
| **1**-cotton (15 wt% **1**) | 56 |
| | |
| nylon | 19 |
| **1**-nylon (15 wt% **1**) | 22 |

| | |
|---|---|
| ^{*a*} Reaction conditions: CH₃CHO (100 ppm) and C₂H₆ (internal standard, 100 ppm) were stirred at 1200 rpm in a 250-mL flask under 1 atm of O₂ at room temperature for 3 hrs. The fabric (0.5g, modified or unmodified with **1** (H₅PV₂Mo₁₀O₄₀) ) was hung in the flask covered with aluminum foil and sealed with a septum stopper. All the values in Table 1 are averages of two experiments. | |
| ^{*b*} Refers to the overall CH₃CHO consumption in the gas phase. | |
| ^{*c*} Refers to the consumption of CH₃CHO by the reaction vessel. | |

### Example 2

**Evaluation of Reusability of the 1-Fabrics in the Aerobic Oxidation of CH**_{**3**}**CHO.** Table 2 illustrates that the reusability of **1**-Smoklin® (5 wt% and 40 wt%) and **1**-acrylic (5 wt%) as catalysts in the aerobic oxidation of CH₃CHO is very high. The catalytic activity of **1**-Smoklin® does not decrease significantly upon the recovery treatment *in vacuo* and reuse.

**Table 2.**

| Reusability of H₅PV₂Mo₁₀O₄₀ (1)-Modified Fabrics in the Removal of CH₃CHO in the Gas Phase.^{*a*} | | | |
|---|---|---|---|
| Fabric | Consumption % of CH₃CHO^{*b*} | | |
| | first use | second use^{*c*} | third use^{*d*} |
| **1**-Smoklin® (5 wt%) | 49 | 40 | 42 |
| **1**-Smoklin® (40 wt%) | 89 | 82 | 83 |
| **1**-acrylic (5 wt%) | 62 | 30 | 32 |

| | | | |
|---|---|---|---|
| ^{*a*} Reaction conditions as described in Table 1. | | | |
| ^{*b*} Refers to the overall CH₃CHO consumption in the gas phase. All values in Table 2 are an average of two experiments. | | | |
| ^{*c*} After the first reaction, the used fabrics were placed *in vacuo* at room temperature for 2 days to remove all adsorbed compounds. The recovered fabrics were then used for a new reaction under the same conditions as in the first run. | | | |
| ^{*d*} The same procedures described in "*c*" were used. | | | |

### Example 3

**Comparison of Catalytic Activities of the 1-Fabrics in the Aerobic Oxidation of CH**_{**3**}**SH.** Table 3 summarizes the catalytic activities of different polyoxometalate-modified fabrics of the present invention in the oxidation of CH₃SH. All of the unmodified fabrics and **1**-cotton cloth (5 wt%) exhibit little or no activity, while **1**-Smoklin® (5 wt%), **1**-acrylic (5 wt%) and **1**-cotton cloth (15 wt%) all possess significant activities. Not wishing to be bound by theory, the high activities observed for the anaerobic reactions involving **1**-Smoklin® (5 wt%) and **1**-acrylic (5 wt%) indicate that 1 most likely functions as a redox catalyst, not simply as a radical chain initiator in the aerobic reactions. The reversible redox chemistry of **1** accounts for the better activity observed in the presence of O₂. In all the reactions containing **1**, dimethyl disulfide (CH₃SSCH₃) is present as the predominant product in gas phase, while in the blank and control reactions, no detectable product exists.

**Table 3.**

| Removal of CH₃SH in the Gas Phase by **1**-Modified Fabrics^{*a*} | | | |
|---|---|---|---|
| Fabric | Consumption % of CH₃SH^{*b*} | | |
| | Step 1 200 ppm^{*c*} at 3hr | Step 2 2000 ppm^{*d*} at 2 hr | Step 3 2000 ppm^{*e*} at 2 hr |
| Blank (O₂)^{*f*} | 36 | 11 | - |
| | | | |
| Smoklin® (O₂) | 30 | 12 | - |
| **1**-Smoklin® (5 wt%, Ar) | 100 | 85 | 14 |
| **1**-Smoklin® (5 wt%,O₂) | 100 | 96 | 47 |
| | | | |
| acrylic (O₂) | 27 | 9 | - |
| **1**-acrylic (5 wt%, Ar) | 100 | 79 | 11 |
| **1**-acrylic (5 wt%,O₂) | 100 | 100 | 100 |
| | | | |
| Cotton (O₂) | 33 | 11 | - |
| **1**-Cotton (5 wt^{%},O₂) | 44 | - | - |
| **1**-Cotton (15 wt%,O₂) | 100 | 52 | 13 (3 hr) |

| | | | |
|---|---|---|---|
| ^{*a*}Reaction conditions: CH₃SH and C₂H₆ (internal standard, 100 ppm) were stirred at 1200 rpm in a 250-mL flask under 1 atm of O₂ or Ar at room temperature. The fabric (0.5g, modified or unmodified with **1**) was hung in the flask covered with aluminum foil and sealed with a septum stopper. All values in Table 3 are averages of two experiments. | | | |
| ^{*b*} Based on the overall CH₃SH consumption in the gas phase. | | | |
| ^{*c*}Step 1: 200 ppm of CH₃SH was added. | | | |
| ^{*d*}Step 2: an additional 2000 ppm of CH₃SH was added after step 1. | | | |
| ^{*e*}Step 3: an additional 2000 ppm of CH₃SH was added after step 2. | | | |
| ^{f} Indicates uptake of CH₃SH by the reaction vessel. | | | |

### Example 4

**Comparison of NH**_{**3**} **Removal Capabilities of the 1-Fabrics.** The results in Table 4 demonstrate that 1 modified Smoklin®, acrylic fiber and cotton cloth are much more active in deodorizing NH₃ than the untreated fabrics. The mole ratios of **1** to NH₃ are 1:3.1 and 1:1 for the reactions of the fabrics modified with 5 wt% and 15 wt% of **1**, respectively. Not wishing to be bound by theory, the mechanism likely involves the acid-base process in equation 1.

H₅PV₂Mo₁₀O₄₀ + xNH₃ → H₅₋ₓPV₂Mo₁₀O₄₀^{-x} + xNH₄⁺ (1)

**Table 4.**

| Removal of NH₃ in the Gas Phase by **1**-Modified Fabrics^{*a*} | |
|---|---|
| Fabric | Consumption% of NH₃^{*b*} |
| Smoklin® | 23 |
| **1**-Smoklin® (5 wt%) | 98 |
| | |
| acrylic | 30 |
| **1**-acrylic (5 wt%) | 97 |
| | |
| cotton | 36 |
| **1**-cotton (5 wt%) | 69 |
| **1**-cotton (15 wt%) | 99 |

| | |
|---|---|
| ^{*a*} Reaction conditions: 200 ppm NH₃ was stirred at 500 rpm in a 5-L flask under 1 atm of Ar at room temperature for 1 hr. The fabric (0.5g, modified or unmodified with 1) was hung in the flask sealed with a septum stopper. All values in Table 4 are averages of two experiments. | |
| ^{*b*} Based on the overall NH₃ consumption in the gas phase. | |

### Example 5

**Comparison of Overall Deodorizing Capabilities of the 1-Fabrics.** The deodorizing efficiencies of **1**-modified fabrics were further evaluated in the contaminated atmosphere simulated by the simultaneous presence of CH₃CHO, CH₃SH and NH₃. The results in Table 5 indicate that **1**-modified Smoklin®, acrylic and cotton cloth are much more effective than the corresponding unmodified fabrics.

**Table 5.**

| Simultaneous Removal of CH₃CHO, CH₃SH and NH₃ in the Gas Phase by **1**-Modified Fabrics^{*a*} | | | |
|---|---|---|---|
| Fabric | Consumption %^{*b*} | | |
| | CH₃CHO | CH₃SH | NH₃ |
| Smoklin® | 31 | 9 | 51 |
| **1**-Smoklin® (5 wt%) | 84 | 68 | 100 |
| | | | |
| acrylic | 27 | 6 | 47 |
| **1**-acrylic (5 wt%) | 63 | 92 | 100 |
| | | | |
| cotton | 33 | 10 | 78 |
| **1**-cotton (15 wt%) | 86 | 66 | 100 |

| | | | |
|---|---|---|---|
| ^{*a*}Reaction conditions: CH₃CHO (100 ppm), CH₃SH (500 ppm), NH₃ (500 ppm) and C₂H₆ (internal standard, 100 ppm) were stirred at 1200 rpm in a 500-mL flask under 1 atm of O₂ at room temperature for 3 hrs. The fabric (0.5g, modified or unmodified with **1**) was hung in the flask covered with aluminum foil and sealed with a septum stopper. All values in Table 5 are averages of two experiments. | | | |
| ^{*b*} Refers to the overall consumption of the reagents in the gas phase. | | | |

The collective data from the gas phase reactions indicate that modifying the cloth or fiber systems with **1** can improve their activity for removing both oxidizable organic compounds by reaction with O₂, and basic compounds by proton donation from the polyoxometalate.

## Claims

1. Use of a polyoxometalate-modified fabric, comprising a fabric and at least one polyoxometalate, wherein the polyoxometalate has the formula [VₖMoₘWₙNbₒTaₚM_{q}XᵣOₛ]^{y-}[A], wherein M is at least one f-block element or d-block element having at least one d-electron, wherein M is not vanadium, molybdenum, tungsten, niobium, or tantalum; X is at least one p-, d-, or f-block element, wherein X is not oxygen; k is from 0 to 30; m is from 0 to 160; n is from 0 to 160; o is from 0 to 10; p is from 0 to 10; q is from 0 to 30; r is from 0 to 30; s is sufficiently large that y is greater than zero; and y is greater than zero, wherein the sum of k, m, n, o, and p is greater than or equal to four; and the sum of k, m, and q is greater than zero, and A is a counterion, wherein the polyoxometalate is incorporated in the fabric,
with the proviso that when A is a proton, the polyoxometalate is not the reaction product between [VₖMOₘWₙNbₒTaₚM_{q}XᵣOₛ]^{y-}[A] and a pararosaniline compound,
with the further proviso that the polyoxometalate is not silicomolybdenic acid or its sodium salt, phosphomolybdenic acid, ammonium chromododecanemolybdenate, ammonium salt of hydrogen hexamolybdocobaltic acid, para-tungstic acid or its ammonium salt or sodium salt, meta-tungstic acid or its ammonium salt or sodium salt, phosphotungstic acid or its salt, silicotungstic acid or its salt, dodecane tungstodicobaltic acid or its salt, phosphotungstomolybdenic acid or its salt, or phosphovanadomolybdenic acid or its salt,
with the further proviso that when the fabric is carbon cloth, the polyoxometalate is not H₅PV₂Mo₁₀O₄₀ for deodorization by removing or degrading malodorous gaseous contaminants.

2. The use of claim 1 for removing offensive odours originating from cigarette smoke, sweat, exhaust gases and rotten food

3. The use of Claim 1, wherein k is greater than or equal to one.

4. The use of Claim 1, wherein the polyoxometalate further comprises an organic group, an organosilyl group, an other p-block organometallic group, or a d-block organometallic group, wherein the organic group, the organosilyl group, the other p-block organometallic group, or the d-block organometallic group is bonded to the polyoxometalate.

5. The use of Claim 1, wherein M comprises titanium, chromium, manganese, cobalt, iron, nickel, copper, rhodium, silver, palladium, platinum, mercury, or ruthenium.

6. The use of Claim 1, wherein X comprises phosphorus, silicon, aluminum, boron, cobalt, zinc, or iron.

7. The use of Claim 1, wherein the polyoxometalate has the formula (X^{g+}V_{b}M^{h+}_{c}Z_{12-b-c}O₄₀]^{u-}[A], wherein X is at least one p-, d-, or f-block element; g+ is the charge of X; M is at least one f-block element or d-block element having at least one d-electron, wherein M is not vanadium; h+ is the charge of M; Z is tungsten, molybdenum, niobium, or a combination thereof; b is from 0 to 6; c is from 0 to 6, wherein the sum of b and c is greater than or equal to one; u is greater than 3; and A is a counterion.

8. The use of Claim 7, wherein the polyoxometalate has the formula [X^{g+}V_{b}Z_{12-b}O_{40]}^{u-}[A], wherein X is at least one phosphorus, silicon, aluminum, boron, zinc, cobalt, or iron; b is from 1 to 6, and u is greater than 3.

9. The use of Claim 1, wherein the polyoxometalate has the structure [X^{g+}M^{h+}_{c}Z_{12-c}O₄₀]^{u-}[A], wherein X^{g+} is at least one phosphorus, silicon, aluminum, boron, zinc, cobalt, or iron; c is from 1 to 6, and u is greater than or equal to 3.

10. The use of Claim 1, wherein the polyoxometalate has the formula [Xⁱ⁺₂VᵤM^{j+}ᵥZ₁₈₋ᵤ₋ᵥO₆₂]^{w-}[A]. wherein X is at least one p-, d-, or f-block element; i+ is the charge of X; M is at least one f-block element or d-block element having at least one d-electron, wherein M is not vanadium; j+ is the charge of M; Z is tungsten, molybdenum, niobium, or a combination thereof; u is from 0 to 9; v is from 0 to 9, wherein the sum of u and v is greater than or equal to one; w is greater than or equal to 4; and A is a counterion.

11. The use of Claim 10, wherein the polyoxometalate has the formula [Xⁱ⁺₂VᵤZ₁₈₋ᵤO₆₂]^{w-}[A], wherein X is at least one phosphorus, sulfur, silicon, aluminum, boron, zinc, cobalt, or iron; u is from 1 to 9; and w is greater than or equal to 4.

12. The use of Claim 10, wherein the polyoxometalate has the formula [Xⁱ⁺₂M^{j+}ᵥZ₁₈₋ᵥO₆₂]^{w-}[A], wherein X is at least one phosphorus, sulfur, silicon, aluminum, boron, zinc, cobalt, or iron; v is from 1 to 9; and w is greater than or equal to 4.

13. The use of Claim 1, wherein the polyoxometalate has the formula [YVₓZ₁₂₋ₓO₄₀][A], wherein Y is phosphorus, silicon, or aluminum; Z is tungsten or molybdenum; x is from 1 to 6, and A is a counterion.

14. The use of Claim 1, wherein the polyoxometalate further comprises an organic group, an organosilyl group, an other p-block organometallic group, or a d-block organometallic group, wherein the organic group, the organosilyl group, the other p-block organometallic group, or the d-block organometallic group is bonded to the polyoxometalate.

15. The use of Claim 1, wherein the polyoxometalate comprises H₅PV₂Mo₁₀O₄₀.

16. The use of Claim 1, wherein the polyoxometalate comprises K₈Co₂W₁₁O₃₉.

17. The use of Claim 1, wherein the fabric is prepared from a fiber comprising polyamide, cotton, polyacrylic, polyacrylonitrile, polyester, polyvinylidine, polyolefin, polyurethane, polytetrafluoroethylene, or carbon cloth, or a combination thereof.

18. The use of Claim 1, wherein the fabric is prepared from a fiber comprising cotton, polyacrylic, or polyacrylonitrile.

19. The use of Claim 1, wherein the fabric is in the form of an article being selected from is garment, drapery, carpet, or upholstery.

20. The use of claim 1 wherein the contaminant comprises an aliphatic nitrogen compound, a sulfur containing compound, an aliphatic oxygenated compound, a halogenated compound, or a combination thereof.

21. The use of Claim 20 wherein the contaminant comprises acetaldehyde, methyl mercaptan, ammonia, hydrogen sulfide, methyl sulfide, dimethyl sulfide, dimethyl disulfide, trimethylamine, styrene, propionic acid, n-butyric acid, n-valeric acid, iso-valeric acid, or a combination thereof.

## Patentansprüche

1. Verwendung eines polyoxometalatmodifizierten Gewebes, das ein Gewebe und mindestens ein Polyoxometalat aufweist, wobei das Polyoxometalat die Formel (VₖMoₘWₙNbₒTaₚM_{q}XᵣOₛ]^{y-}[A] hat, worin M mindestens ein f-Blockelement oder d-Blockelement mit mindestens einem d-Elektron ist, wobei M nicht Vanadium, Molybdän, Wolfram, Niob oder Tantal ist; X mindestens ein p-, d- oder f-Blockelement ist, wobei X nicht Sauerstoff ist; k 0 bis 30 ist; m 0 bis 160 ist; n 0 bis 160 ist; o 0 bis 10 ist; p 0 bis 10 ist; q 0 bis 30 ist; r 0 bis 30 ist; s ausreichend groß ist, so dass y größer null ist und y größer als null ist, wobei die Summe von k, m, n, o und p größer oder gleich vier ist und die Summe von k, m und q größer als null ist und A ein Gegenion ist, wobei das Polyoxometalat in das Gewebe eingearbeitet ist
mit dem Vorbehalt, dass dann, wenn A ein Proton ist, das Polyoxometalat nicht das Reaktionsprodukt von [VₖMoₘWₙNbₒTaₚM_{q}XᵣOₛ]^{y-}[A] und einer Pararosanilinverbindung ist,
mit dem weiteren Vorbehalt, dass das Polyoxometalat nicht Silicomolybdänsäure oder ihr Natriumsalz, Phosphomolybdänsäure, Ammoniumchromdodecanmolybdat, das Ammoniumsalz von Hydrogenhexamolybdocobaltsäure, Parawolframsäure oder das Ammoniumsalz oder Natriumsalz davon, Metawolframsäure oder das Ammoniumsalz oder Natriumsalz davon, Phosphowolframsäure oder das Salz davon, Silicowolframsäure oder das Salz davon, Dodecanwolframdicobaltsäure oder das Salz davon, Phosphowolframmolybdänsäure oder das Salz davon oder Phosphovanadomolybdänsäure oder das Salz davon ist,
mit dem weiteren Vorbehalt, dass dann, wenn das Gewebe Kohlenstofftuch ist, das Polyoxometalat nicht H₅PV₂Mo₁₀O₄₀ ist, zur Desodorisierung durch Entfernung oder Abbau von übel riechenden gasförmigen Kontaminanten.

2. Verwendung nach Anspruch 1 zur Entfernung aufdringlicher Gerüche, die aus Zigarettenrauch, Schweiß, Abgasen und verdorbenen Lebensmitteln entstehen.

3. Verwendung nach Anspruch 1, wobei k größer oder gleich eins ist.

4. Verwendung nach Anspruch 1, wobei das Polyoxometalat weiterhin eine organische Gruppe, eine Organosilylgruppe, eine andere p-Block-organometallische Gruppe oder eine d-Block-organometallische Gruppe aufweist, wobei die organische Gruppe, die Organosilylgruppe, die andere p-Block-organometallische Gruppe oder d-Block-organometallische Gruppe an das Polyoxometalat gebunden ist.

5. Verwendung nach Anspruch 1, wobei M Titan, Chrom, Mangan, Cobalt, Eisen, Nickel, Kupfer, Rhodium, Silber, Palladium, Platin, Quecksilber oder Ruthenium aufweist.

6. Verwendung nach Anspruch 1, wobei X Phosphor, Silicium, Aluminium, Bor, Cobalt, Zink oder Eisen aufweist.

7. Verwendung nach Anspruch 1, wobei das Polyoxometalat die Formel [X^{g+}V_{b}M^{h+}_{c}Z_{12-b-c}O₄₀]^{u-}[A] hat, worin X mindestens ein p-, d- oder f-Blockelement ist; g+ die Ladung von X ist; M mindestens ein f-Blockelement oder d-Blockelement mit mindestens einem d-Elektron ist, wobei M nicht Vanadium ist; h+ die Ladung von M ist; Z Wolfram, Molybdän, Niob oder eine Kombination davon ist; b 0 bis 6 ist; c 0 bis 6 ist; wobei die Summe von b und c größer oder gleich 1 ist; u größer 3 ist und A ein Gegenion ist.

8. Verwendung nach Anspruch 7, wobei das Polyoxometalat die Formel [X^{g+}V_{b}Z_{12-b}O₄₀]^{u-}[A] hat, worin X mindestens ein Phosphor, Silicium, Aluminium, Bor, Zink, Cobalt oder Eisen ist; b 1 bis 6 ist und u größer 3 ist.

9. Verwendung nach Anspruch 1, wobei das Polyoxometalat die Struktur [X^{g+}M^{h+}_{c}Z_{12-c}O₄₀]^{u-}[A] hat, wobei X^{g+} mindestens ein Phosphor, Silicium, Aluminium, Bor, Zink, Cobalt oder Eisen ist; c 1 bis 6 ist und u größer oder gleich 3 ist.

10. Verwendung nach Anspruch 1, wobei das Polyoxometalat die Formel [Xⁱ⁺₂VᵤM^{j+}ᵥZ₁₈₋ᵤ₋ᵥO₆₂]^{w-}[A] hat, worin X mindestens ein p-, d- oder f-Blockelement ist; i+ die Ladung von X ist; M mindestens ein f-Blockelement oder d-Blockelement mit mindestens einem d-Elektron ist, wobei M nicht Vanadium ist; j+ die Ladung von M ist; Z Wolfram, Molybdän, Niob oder eine Kombination davon ist; u 0 bis 9 ist; v 0 bis 9 ist, wobei die Summe von u und v größer oder gleich 1 ist; w größer oder gleich 4 ist und A ein Gegenion ist.

11. Verwendung nach Anspruch 10, wobei das Polyoxometalat die Formel [Xⁱ⁺₂VᵤZ₁₈₋ᵤO₆₂]^{w-}[A] hat, wobei X mindestens ein Phosphor, Schwefel, Silicium, Aluminium, Bor, Zink, Cobalt oder Eisen ist; u 1 bis 9 ist und w größer oder gleich 4 ist.

12. Verwendung nach Anspruch 10, wobei das Polyoxometalat die Formel [Xⁱ⁺₂M^{j+}ᵥZ₁₈₋ᵥO₆₂]^{w-}[A] hat, wobei X mindestens ein Phosphor, Schwefel, Silicium, Aluminium, Bor, Zink, Cobalt oder Eisen ist; v 1 bis 9 ist und w größer oder gleich 4 ist.

13. Verwendung nach Anspruch 1, wobei das Polyoxometalat die Formel [YVₓZ₁₂₋ₓO₄₀][A] hat, wobei Y Phosphor, Silicium oder Aluminium ist; Z Wolfram oder Molybdän ist; x 1 bis 6 ist und A ein Gegenion ist.

14. Verwendung nach Anspruch 1, wobei das Polyoxometalat weiterhin eine organische Gruppe, eine Organosilylgruppe, eine weitere p-Block-organometallische Gruppe oder eine d-Block-organometallische Gruppe aufweist, wobei die organische Gruppe, die Organosilylgruppe, die andere p-Block-organometallische Gruppe oder die d-Block-organometallische Gruppe an das Polyoxometalat gebunden ist.

15. Verwendung nach Anspruch 1, wobei das Polyoxometalat H₅PV₂Mo₁₀O₄₀ beinhaltet.

16. Verwendung nach Anspruch 1, wobei das Polyoxometalat K₈Co₂W₁₁O₃₉ beinhaltet.

17. Verwendung nach Anspruch 1, wobei das Gewebe aus einer Faser, die Polyamid, Baumwolle, Polyacryl, Polyacrylnitril, Polyester, Polyvinylidin, Polyolefin, Polyurethan, Polytetrafluorethylen enthält, oder Kohlenstofftuch oder einer Kombination davon hergestellt wird.

18. Verwendung nach Anspruch 1, wobei das Gewebe aus einer Faser hergestellt wird, die Baumwolle, Polyacryl oder Polyacrylnitril enthält.

19. Verwendung nach Anspruch 1, wobei das Gewebe in Form eines Gegenstands ist, der ausgewählt ist aus Kleidungsstücken, Textilien, Teppichen oder Polsterwaren.

20. Verwendung nach Anspruch 1, wobei das kontaminierende Mittel eine aliphatische Stickstoffverbindung, eine schwefelhaltige Verbindung, eine aliphatische sauerstoffhaltige Verbindung, eine halogenierte Verbindung oder eine Kombination davon aufweist.

21. Verwendung nach Anspruch 20, wobei das kontaminierende Mittel Acetaldehyd, Methylmercaptan, Ammoniak, Schwefelwasserstoff, Methylsulfid, Dimethylsulfid, Dimethyldisulfid, Trimethylamin, Styrol, Propionsäure, n-Buttersäure, n-Valeriansäure, Isovaleriansäure oder eine Kombination davon aufweist.

## Revendications

1. Utilisation d'un tissu modifié par un polyoxométallate, comprenant un tissu et au moins un polyoxométallate, où le polyoxométallate présente la formule [VₖMoₘWₙNbₒTaₚM_{q}XᵣOₛ]^{y-}[A], où M est au moins un élément du bloc f ou un élément du bloc d comportant au moins un électron d, où M n'est pas le vanadium, le molybdène, le tungstène, le niobium ni le tantale, X est au moins un élément du bloc p, d ou f, où X n'est pas l'oxygène, k vaut de 0 à 30, m vaut de 0 à 160, n vaut de 0 à 160, o vaut de 0 à 10, p vaut de 0 à 10, q vaut de 0 à 30, r vaut de 0 à 30, s est suffisamment grand pour que y soit supérieur à zéro, et y est supérieur à zéro, où la somme de k, m, n, o et p est supérieur ou égal à quatre, et la somme de k, m et q est supérieur à zéro et A est un contre-ion, où le polyoxométallate est incorporé dans le tissu,
à la condition que, lorsque A est un proton, le polyoxométallate ne soit pas le produit de réaction entre (VₖMoₘWₙNbₒTaₚM_{q}XᵣOₛ]^{y-}[A] et un composé de pararosoaniline,
avec l'autre condition que le polyoxométallate ne soit pas l'acide silicomolybdènique ni son sel de sodium, l'acide phosphomolybdènique, du chromododécanemolybdénate d'ammonium, un sel d'ammonium d'acide hydrogéno hexamolybdocobaltique, d'acide para-tungstique ni son sel d'ammonium, ni son sel de sodium, d'acide métatungstique ni son sel d'ammonium, ni son sel de sodium, d'acide phosphotungstique ni son sel, d'acide silcotungstique ni son sel, d'acide dodécane tungstodicobaltique ni son sel, d'acide phosphotungstomolybdénique ni son sel, ni d'acide phosphovanadomolybdénique ni son sel,
avec la condition supplémentaire que lorsque le tissu est une étoffe de carbone, le polyoxométallate n'est pas du H₅PV₂Mo₁₀O₄₀ pour la désodorisation par l'élimination ou la dégradation de contaminants gazeux mal odorants.

2. Utilisation selon la revendication 1 destinée à éliminer les odeurs agressives ayant pour origine la fumée de cigarettes, la sueur, les gaz d'échappement et les aliments avariés.

3. Utilisation selon la revendication 1, dans laquelle k est supérieur ou égal à 1.

4. Utilisation selon la revendication 1, dans laquelle le polyoxométallate comprend en outre un groupement organique, un groupement organosilyle, un autre groupement organométallique du bloc p ou un groupement organométallique du bloc d où le groupement organique, le groupement organosilyle, l'autre groupement organométallique du groupe p ou le groupement organométallique du bloc d est lié au polyoxométallate.

5. Utilisation selon la revendication 1, dans laquelle M comprend le titane, le chrome, le manganèse, le cobalt, le fer, le nickel, le cuivre, le rhodium, l'argent, le palladium, le platine, le mercure, ou le ruthénium.

6. Utilisation selon la revendication 1, dans laquelle X comprend du phosphore, du silicium, de l'aluminium, du bore, du cobalt, du zinc ou du fer.

7. Utilisation selon la revendication 1, dans laquelle le polyoxométallate présente la formule [X^{g+}V_{b}M^{h+}_{c}Z_{12-b-c}O₄₀]^{u-}[A], où X est au moins un élément du bloc p, d ou f, g+ représente la charge de X, M est au moins un élément du bloc f ou un élément du bloc d comportant au moins un électron d, où M n'est pas le vanadium, h+ représente la charge de M, Z est le tungstène, le molybdène, le niobium ou une combinaison de ceux-ci, b vaut de 0 à 6, c vaut de 0 à 6, où la somme de b et c est supérieure ou égale à un, u est supérieur à 3, et A est un contre-ion.

8. Utilisation selon la revendication 7, dans laquelle le polyoxométallate présente la formule [X^{g+}V_{b}Z_{12-b-c}O₄₀]^{u-}[A] où X est au moins l'un du phosphore, du silicium, de l'aluminium, du bore, du zinc, du cobalt, ou du fer, b vaut de 1 à 6 et u est supérieur à 3.

9. Utilisation selon la revendication 1, dans laquelle le polyoxométallate présente la structure [X^{g+}M^{h+}_{c}Z_{12-c}O₄₀]^{u-}[A], où X^{g+} est au moins l'un du phosphore, du silicium, de l'aluminium, du bore, du zinc, du cobalt ou du fer, c vaut de 1 à 6 et u est supérieur ou égal à 3.

10. Utilisation selon la revendication 1, dans laquelle le polyoxométallate présente la formule [Xⁱ⁺₂VᵤMⁱ⁺ᵥZ₁₈₋ᵤ₋ᵥO₆₂]^{w-}[A], où X est au moins un élément du bloc p, d ou f, i+ représente la charge de X, M est au moins un élément du bloc f ou un élément du bloc d ayant au moins un électron d, où M n'est pas le vanadium, j+ représente la charge de M, Z est le tungstène, le molybdène, le niobium ou une combinaison de ceux-ci, u vaut de 0 à 9, v vaut de 0 à 9, où la somme de u et v est supérieure ou égale à un, w est supérieur ou égal à 4, et A est un contre-ion.

11. Utilisation selon la revendication 10, dans laquelle le polyoxométallate présente la formule [Xⁱ⁺₂VᵤZ₁₈₋ᵤO₆₂]^{w-}[A], dans laquelle X est au moins l'un du phosphore, du soufre, du silicium, de l'aluminium, du bore, du zinc, du cobalt ou du fer, u vaut de 1 à 9, et w est supérieur ou égal à 4.

12. Utilisation selon la revendication 10, dans laquelle le polyoxométallate présente la formule [Xⁱ⁺₂M^{j+}ᵥZ₁₈₋ᵥO₆₂]^{w-}[A], où X est au moins l'un du phosphore, du soufre, du silicium, de l'aluminium, du bore, du zinc, du cobalt ou du fer, v vaut de 1 à 9, et w est supérieur ou égal à 4.

13. Utilisation selon la revendication 1, dans laquelle le polyoxométallate présente la formule [YVₓZ₁₂₋ₓO₄₀][A], où Y est le phosphore, le silicium ou l'aluminium, Z est le tungstène ou le molybdène, x vaut de 1 à 6, et A est un contre-ion.

14. Utilisation selon la revendication 1, dans laquelle le polyoxométallate comprend en outre un groupement organique, un groupement organosilyle, un autre groupement organométallique du bloc p, ou un groupement organométallique du bloc d, dans lequel le groupement organique, le groupement organosilyle, l'autre groupement organométallique du bloc p, ou le groupement organométallique du bloc d est lié au polyoxométallate.

15. Utilisation selon la revendication 1, dans laquelle le polyoxométallate comprend du H₅PV₂Mo₁₀O₄₀.

16. Utilisation selon la revendication 1, dans laquelle le polyoxométallate comprend du K₈Co₂W₁₁O₃₉.

17. Utilisation selon la revendication 1, dans laquelle le tissu est préparé à partir d'une fibre comprenant du polyamide, du coton, un polyacrylique, un polyacrylonitrile, un polyester, un polyvinylidène, une polyoléfine, un polyuréthane, un polytétrafluoroéthylène ou un tissu de carbone ou bien une combinaison de ceux-ci.

18. Utilisation selon la revendication 1, dans laquelle le tissu est préparé à partir d'une fibre comprenant du coton, un polyacrylique ou un polyacrylonitrile.

19. Utilisation selon la revendication 1, dans laquelle le tissu est sous forme d'un article qui est choisi parmi un vêtement, une étoffe, un tapis ou une tapisserie.

20. Utilisation selon la revendication 1, dans laquelle le contaminant comprend un composé d'azote aliphatique, un composé contenant du soufre, un composé aliphatique oxygéné, un composé halogéné ou une combinaison de ceux-ci.

21. Utilisation selon la revendication 20, dans laquelle le contaminant comprend de l'acétaldéhyde, du méthyle mercaptan, de l'ammoniac, du sulfure d'hydrogène, du sulfure de méthyle, du sulfure de diméthyle, du disulfure de diméthyle, de la triméthylamine, du styrène, de l'acide propionique, de l'acide n-butyrique, de l'acide n-valérique, de l'acide iso-valérique, ou une combinaison de ceux-ci.
